# EUROPEAN PATENT APPLICATION

(11) **EP 4 741 825 A1**
(43) Date of publication of application: **13.05.2026**
(21) Application number: 24839616.0
(22) Date of filing: 03.07.2024
(51) Int. Cl.: G01N 33/543, G01N 21/64

(54) **TEST KIT**

(30) Priority: 07.07.2023 JP 2023112292
(71) Applicant: Lifetrek Inc., Tokyo 103-0016 (JP)
(72) Inventor: SHIBASAKI Futoshi, Tokyo 103-0016 (JP); KAJIWARA Naoki, Tokyo 103-0016 (JP)
(74) Representative: Vossius & Partner Patentanwälte Rechtsanwälte mbB
(86) International application number: PCT/JP2024/024090
(87) International publication number: WO 2025/013724

(57) **Abstract**

[Problem]

To provide a test kit that does not require a complicated device to perform detection and that allows easier detection confirmation than conventional test kits in which chromatography is used.

[Solution]

Provided is a test kit including: a sample pad that absorbs a sample; a conjugate pad that has a detection marker supported thereon; a chromatographic body that has a membrane; an absorption pad that prevents backflow of the sample; and a light source that irradiates light of 300 nm to 400 nm. The test kit is characterized in that the detection marker is a fluorescent substance that emits light having a wavelength of at least 600 nm when light having a wavelength of 300 nm to 400 nm is applied.

## Description

### Technical Field

The present invention relates to a test kit for detecting various target objects such as an antibody, an antigen, a protein, a biomarker, a chemical molecule, and a nucleic acid. More specifically, the present invention relates to a test kit that does not require a complicated device for detection and allows for easier detection and confirmation compared to conventional chromatography-based test kits.

### Background Art

A large number of test kits for detecting various target objects, such as an antibody, an antigen, a protein, a biomarker, a chemical molecule, and a nucleic acid, have been proposed. Among these, test kits using immunochromatography are widely used due to the ease of use thereof, and various types thereof have been proposed.

For example, PTL 1 proposes an immunochromatography test piece that is capable of achieving both highly sensitive detection of a substance to be detected and a simple test piece structure, which are generally mutually exclusive. Specifically, an immunochromatographic test piece has been proposed, wherein the immunochromatographic test piece is an immunochromatographic test piece for measuring a substance to be detected, by: including therein a membrane on which a capture substance is immobilized, which is a ligand that binds to a substance to be detected; using insoluble carrier particles to which a ligand that binds to a substance to be detected is bound; accumulating the insoluble carrier particles by capturing the same with the capture substance immobilized on the membrane; and irradiating the membrane with light to detect light emitted at a site where the insoluble carrier particles have accumulated or at a surrounding site other than the site where the insoluble carrier particles have accumulated, and wherein the immunochromatographic test piece includes a light-reflecting material on a side opposite to the side, where the light is applied, of the membrane.

In addition, PTL 2 proposes a test kit that has excellent detection sensitivity for a substance to be detected. Specifically, a test kit has been proposed, wherein the test kit has: on a base material, a reagent retaining portion containing a labeling reagent in which a binding substance that specifically binds to a substance to be detected is immobilized on labeled fine particles; and a test part on which a test capture substance that specifically binds to a substance to be detected is immobilized, and the labeled fine particles are fine particles made of a noble metal, and the test part contains an up-conversion material.

In addition, PTL 3 discloses a test kit, wherein the test kit is a test kit for detecting and/or quantifying a biological or chemical substance of interest in a liquid sample with a capillary action test by using, as a probe, photoluminescent inorganic nanoparticles of Formula A1 - xLnxVO4 (1-y) (PO4)y (II), where in the formula, Ln is selected from europium (Eu), dysprosium (Dy), samarium (Sm), neodymium (Nd), erbium (Er), ytterbium (Yb), thulium (Tm), praseodymium (Pr), holmium (Ho), and a mixture thereof, A is selected from yttrium (Y), gadolinium (Gd), lanthanum (La), lutetium (Lu), and a mixture thereof, 0 < x < 1 and 0 ≤ y <1 are satisfied, and moreover the method thereof detects emission of nanoparticles having an emission lifetime of less than 100 ms after one-photon absorption by exciting a matrix at a wavelength of 320 nm or less.

### Citation List

### Patent Literature

PTL 1: JP No. 2015-31610A
PTL 2: JP No. 2017-223616A
PTL 3: JP No. 2021-530700A

### Summary of Invention

### Technical Problem

However, in the test kit proposed above, the chromatographic results cannot be clearly confirmed by visual inspection(observation), and thus it is necessary to use a detection device for reading. This poses a problem in that detection and confirmation cannot be easily carried out due to , for instance, the requirement for a complicated device.

As a result, an object of the present invention is to provide a test kit that does not require a complicated device for detection and allows detection and confirmation to be implemented easier, as compared with conventional chromatography-based test kits.

### Solution to Problem

As a result of carrying out intensive studies to solve the above-described problems, the inventors have found that when a europium complex is used for a target reagent, detection and confirmation can be carried out by visual inspection(observation) using only ultraviolet light (such as a commonly used so-called "black light") without a special device. and as a result of carrying out further studies based on such finding, the inventors of the present invention have completed the present invention.

That is, the present invention provides each of the following inventions.
1. A test kit comprising:
   a sample pad that absorbs a specimen;
   a conjugate pad on which a detection marker is supported;
   a chromatographic body comprising a membrane;
   an absorption pad that prevent backflow of the specimen; and
   a light source configured to emit light of 300 nm to 400 nm,
   wherein the detection marker contains a fluorescent substance that emits light having a wavelength of 600 nm or more in a case of being irradiated with light having a wavelength of 300 nm to 400 nm.
2. The test kit according to 1, wherein the fluorescent substance is a rare earth complex.
3. The test kit according to 1, wherein the fluorescent substance has a fluorescence brightness of 0.8 or more when a brightness of an excitation light is set to 1.
4. The test kit according to 1, further comprising:
   a fluorescent color level sample,
   wherein the test kit is configured to allow quantification by visually observing and confirming a color level of the fluorescent color level sample and a chromatographic fluorescent color.
5. The test kit according to 2, wherein the fluorescent substance is a polymer particle encapsulating t the rare earth complex.
6. The test kit according to 5, wherein a mixing ratio of the polymer particle is 20 to 80 in terms of detection antibody/particle (mg/g) in the detection marker.

The test kit according to the present invention does not require a complicated device for detection. And the test kit allows detection and confirmation to be implemented easier, as compared with conventional chromatography-based test kits. Therefore, the test kit according to the present invention is excellent in terms of cost and is useful as a test kit for detecting various target objects such as an antibody, an antigen, a protein, a biomarker, a chemical molecule, and a nucleic acid.

Further, the test kit according to the present invention is a test kit that has excellent confidentiality because the confirmation can be carried out only after irradiation with light having a specific wavelength. In addition, detection can be carried out with high sensitivity as compared with conventional immunochromatography. Specifically, by using a fluorescent dye such as europium, detection can be carried out at a fluorescence wavelength (615 nm) that is farther from the excitation light wavelength (360 to 370 nm) of black light. Therefore, it is possible to carry out detection with a high a signal/noise (S/N) ratio and high sensitivity, as compared with conventional labels such as gold colloids (reduced background light due to the Stokes shift). In addition, further higher sensitivity is expected under conditions where there is no external light, such as in a darkroom, or conditions where light is blocked.

### Brief Description of Drawings

[Fig. 1]

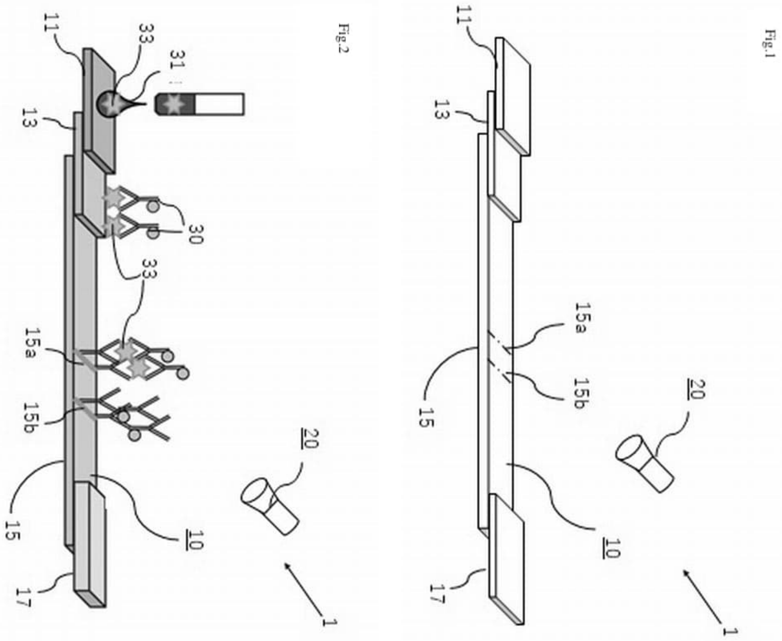

   Fig. 1 is a schematic view illustrating an overall configuration of a test kit according to the present invention.
[Fig. 2]
   Fig. 2 is an explanatory view schematically illustrating an use embodiment of the test kit illustrated in Fig. 1.
[Fig. 3]

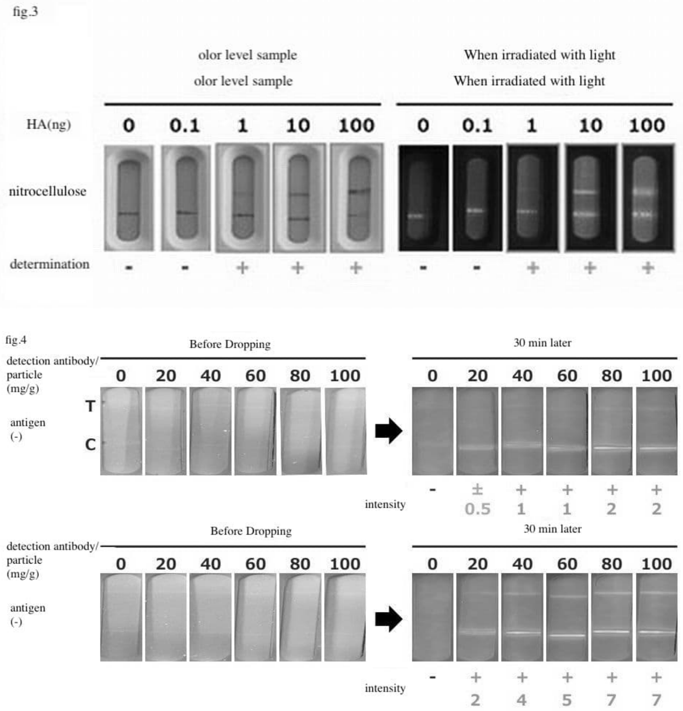

   Fig. 3 is a photographic image (a photographic image substituting for the drawing) showing a case where a detection kit of Example is used.
[Fig. 4]
   Fig. 4 is a photographic image (a photographic image substituting for the drawing) showing the results of using a detection kit of Example 2.
[Fig. 5]

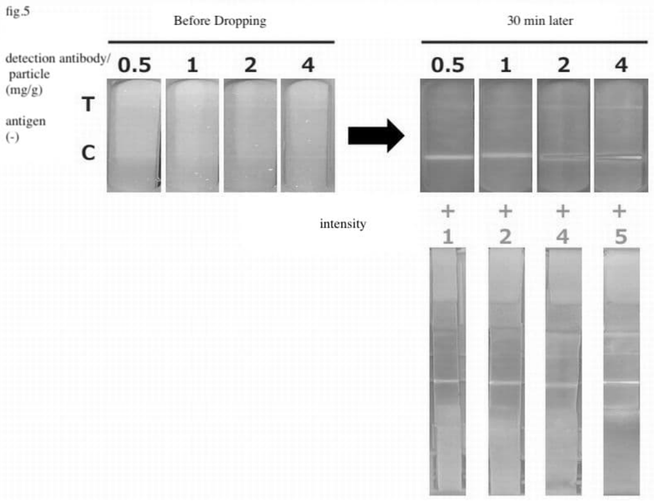

   Fig. 5 is a photographic image (a photographic image substituting for the drawing) showing a case where the detection kit of Example 2 is used in another embodiment.
[Fig. 6]

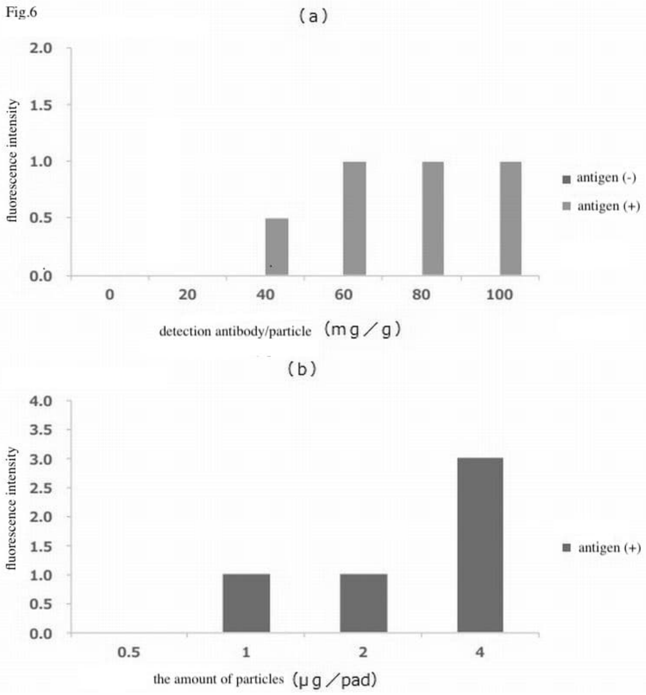

   Fig. 6 is a chart showing the results of using the detection kit of Example 2, where (a) is a chart showing the relationship between detection antibody/particles and fluorescence intensity, and (b) is a chart showing the relationship between the amount of particles and fluorescence intensity.
[Fig. 7]

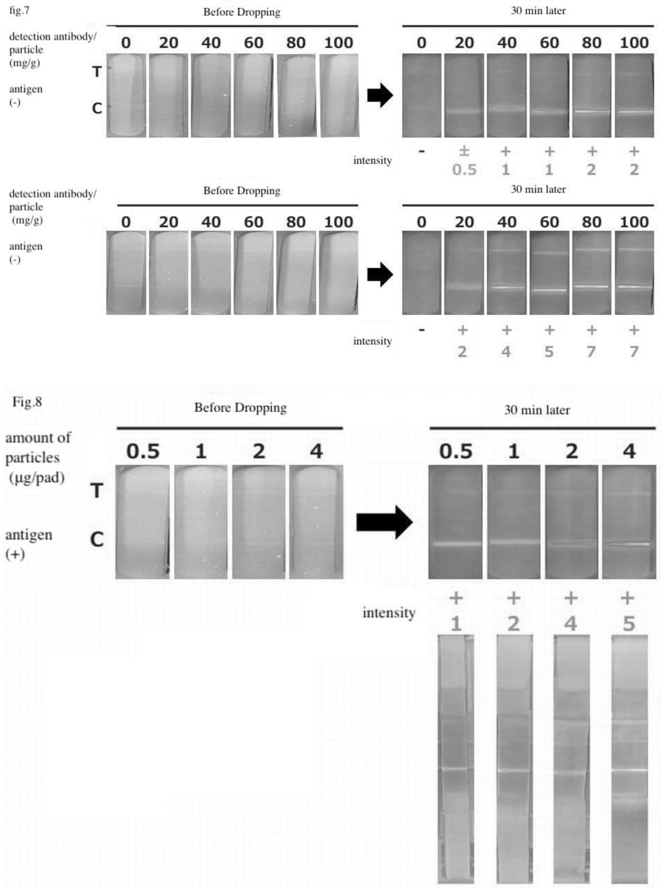

   Fig. 7 is a photographic image (a photographic image substituting for the drawing) showing the results of using a detection kit of Example 3.
[Fig. 8]
   Fig. 8 is a photographic image (a photographic image substituting for the drawing) showing a case where the detection kit of Example 3 is used in another embodiment.
[Fig. 9]

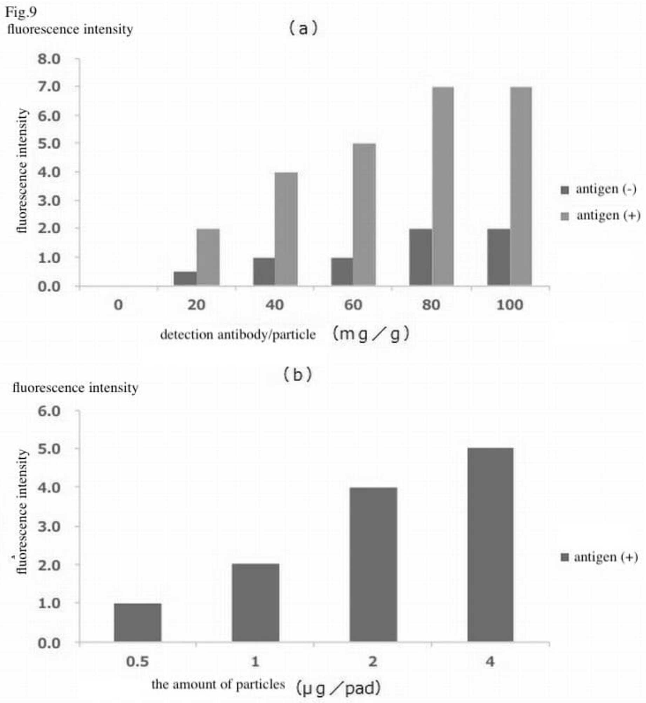

   Fig. 9 is a chart showing the results of using the detection kit of Example 3, where (a) is a chart showing the relationship between detection antibody/particles and fluorescence intensity, and (b) is a chart showing the relationship between the amount of particles and fluorescence intensity.

### Reference Signs List

- 1: Test kit
- 10: Chromatographic body
- 11: Sample pad
- 13: Conjugate pad
- 15: Membrane
- 17: Absorption pad
- 20: Light source

### Description of Embodiments

Hereinafter, the present invention will be described in further detail.

As illustrated in Fig. 1, a test kit 1 according to one embodiment of the present invention includes a chromatographic body 10 and a light source 20. The chromatographic body 10 has a sample pad 11 that absorbs a specimen, a conjugate pad 13 on which a detection marker is supported, an absorption pad 17 that absorbs an electrophoresed specimen and prevents backflow, and a membrane 15. And a light source 20 that emits light of 300 nm to 400 nm.

Here, the term "detection marker" refers to a mixture of a detection antibody and a fluorescent substance (including both a mixture in which a detection antibody is bound to a fluorescent substance and a mixture in which a detection antibody is not bound to a fluorescent substance).

Further details are described below.

### [Detection Target]

The detection target in the detection kit according to the present embodiment includes those that can serve as a test target as an antibody, an antigen, a protein, a nucleic acid, and another biomarker other than these, as well as other compounds and the like.

### [Chromatographic Body]

The chromatographic body 10 includes the membrane 15, which is a member for separating and visualizing members, which are the main functions of chromatography; the conjugate pad 13 attached with a detection marker 30 (see Fig. 2) attached thereto and the absorption pad 17 for absorbing excess liquid, which are respectively placed on both end portions of the membrane 15 in the longitudinal direction; and the sample pad 11 which is placed on the end portion of the conjugate pad 13. In addition, a test line 15a and a control line 15b are provided in the membrane 15 in accordance with the conventional method. These members can be formed to have the same shape and thickness as those of the members that are used in general immunochromatography. In the present embodiment, all of the shapes are rectangular shapes, and the thickness is allowed to adopt a thickness that can be usually adopted, without any particular limitation.

For the materials for forming these members, materials that are generally used for this type of chromatography can be used without any particular limitation. In the present embodiment, the membrane 15 is formed from a nonwoven fabric such as a polyester nonwoven fabric, and the conjugate pad 13, the absorption pad 17, and the sample pad 11 are formed from a nonwoven fabric made of cellulose fibers. It is noted that the conjugate pad may be made of glass fiber.

In addition, although not particularly illustrated, the chromatographic body according to the present embodiment may include a case or a cover, which covers the above-described members. In addition, a member such as a fluorescent filter or a band pass filter, which is advantageous for Visual inspection(observation), may be provided.

It is noted that although a chromatographic body having two types of lines is shown in the present embodiment, the present invention is not limited thereto, and three or more types of lines may be provided.

In addition, instead of the rectangular shape as in the present embodiment, various shapes such as a circular shape, can be adopted.

As the material for forming the membrane, in addition to the nonwoven fabric described above, a material that is generally used to form this type of membrane which is made of a metal or the like can be used without any particular limitation.

### [Detection Marker]

In the present embodiment, the detection marker supported on the conjugate pad 13 contains a fluorescent substance that emits light having a wavelength of 600 nm or more, preferably light having a wavelength of 500 to 700 nm (hereinafter, may be referred to as "fluorescence") in a case of being exposed to light having a wavelength of 300 nm to 400 nm (hereinafter, may be referred to as "absorption light"). That is, the detection marker is a mixture containing a detection antibody and a fluorescent substance. By using a detection marker containing such a fluorescent substance, it is possible to carry out detection through Visual inspection(observation) by using a light source such as a commercially available fluorescent lamp, as described below, without using a special detection device.

In the conventional immunochromatography, emphasis has been placed on the sensitivity of emission, and even in research reports and the like using fluorescent substances, all of them have merely aimed to increase sensitivity, and there have been no reports that focus on the Stokes shift in fluorescence (the difference between the maximum excitation wavelength and the maximum wavelength of emitted light (light emitted from a fluorescent substance)). The inventors have found that a narrow Strokes shift generates background noise, which makes confirmation by visual inspection(observation) difficult, and have found that by widening the Stokes shift, detection and confirmation by visual inspection (observation) become sufficiently possible without a special device. Therefore, the wavelength of the absorption light and the wavelength of the emitted light are important, and it is important to use a fluorescent substance having a wide Stokes shift.

The fluorescence brightness of the fluorescent substance (the brightness at the wavelength where fluorescence is emitted) is preferably 0.8 or more and more preferably in a range of 0.9 to 1.5 in terms of and confirming by visually observing the light from the fluorescent substance in a case where the brightness of the excitation light (light at the maximum excitation wavelength) is set to 1.

Specific examples of the fluorescent substance include a rare earth fluorescent complex and the like, and examples of the rare earth complex include a europium complex, a terbium complex, a samarium complex, and a dysprosium complex. Among these, particularly one or more compounds selected from the group consisting of an europium complex, a terbium complex, a samarium complex, and a dysprosium complex can be preferably used as the fluorescent substance. Among these, a europium complex is preferable in terms of obtaining a desired effect of the present invention, and specific examples of the europium complex include the following compounds.

Examples thereof include:
sodium [4'-(4'-amino-4-biphenylyl)-2,2':6',2"-terpyridine-6,6"-diylbis(methyliminodiacetate)]europate(III) (ATBTA-Eu³⁺),
Eu³⁺ complex of N¹-(p-isothiocyanatobenzyl)-ethylenediaminetetraacetic acid (SCN-Ph-EDTA-Eu³⁺),
Eu³⁺ complex of N¹-(p-isothiocyanatobenzyl)-diethylenetriamine-N¹,N²,N³,N³-tetraacetic acid (SCN-Ph-DTTA-Eu³⁺),
Eu(β-NTA)₃(TOPO)₂ (having a detection limit at a level of 10⁻¹⁴ M): it is necessary to add a so-called fluorescence enhancement solution containing β-NTA-TOPO-Triton X-100,
a chlorosulfonylated tetradentate β-diketone compound 4,4'-bis(1",1",1",2",2",3",3"-heptafluoro-4",6"-hexanedione-6"-yl)chlorosulfo-o-terphenyl (BHHCT) BHHCT-Eu³⁺, which uses, as a labeling agent, Eu³⁺ complex of 4,7-bis(chlorosulfophenyl)-1,10-phenanthroline-2,9-dicarboxylic acid (BCPDA-Eu³⁺),
a ternary complex of an EDTA-Tb³⁺ complex and a salicylic acid derivative,
a combined use of two kinds of fluorescent labeling agents, tris bipyridine cryptate-Eu³⁺ (a donor dye for fluorescence energy transfer, abbreviated as TBP-Eu³⁺) and allophycocyanin (an acceptor dye for fluorescence energy transfer, a cross-linked allophycocyanin, a dye protein having a molecular weight of 104 kD, maximum fluorescence emission wavelength: 665 nm, fluorescence quantum yield: approximately 0.7, abbreviated as XL665),
BCPDA-Eu³⁺ labeled polyvinylamine-biotin-streptavidin complex,
a combined use of a poly(Glu:Lys)-streptavidin conjugate and a europium fluorescent complex-labeled poly(Glu:Lys)-BSA-streptavidin conjugate,
a complex of 4-[2-(4-isothiocyanatophenyl)ethynyl]-2,6-bis{[N,N-bis(carboxymethyl)amino]methyl}pyridine and Eu³⁺,
those in a state of being encapsulated in particles, for example, polystyrene latex fine nanoparticles containing a europium fluorescent complex,
and the like.

In addition, the following commercially available products can also be used.
Estapor [registered trademark] europium microsphere series (manufactured by Merck KGaA)
Europium Conjugation Kit (ab269889, manufactured by Abcam plc)
FS Eu beads (manufactured by TAMAGAWA SEIKI CO., LTD.), and the like.

The mixing amount of the fluorescent substance may be set to any amount depending on the detection target; however, it is preferable to use the fluorescent substance by adjusting the amount so that the number of fluorescent substances is in a range of 1 to 20 and particularly preferably in a range of 8 to 12 with respect to one detection target. This adjustment is determined depending on the expected amount of the detection target (calculated from the predicted content of the detection target with respect to the collected amount of the specimen).

In addition, the above-described complex or the like can be used as it is as the fluorescent substance. Further, it is preferable to use the above-described complex in a state of being encapsulated in particles, from the viewpoint of dispersibility in the membrane and visibility of the fluorescence by Visual inspection. For example, it is preferable to use the above-described commercially available product Estapor (registered trademark) europium microsphere series (manufactured by Merck KGaA, see Examples).

The mixing ratio of the detection antibody to the fluorescent substance is not particularly limited; however, the mixing ratio of detection antibody/particle (mg/g) is preferably set to 20 to 80, more preferably set to 40 to 80, and most preferably set to 40 to 60 in a case where particles (polymer particles and the like) encapsulating the above-described complex are used.

As the detection antibody used in the detection marker, an antibody that is generally used for the detection marker in this type of immunochromatography can be used without any particular limitation.

Examples thereof include an anti-H5 HA monoclonal antibody (5H7) and the like.

### [Light Source]

The light source used in the present embodiment is not particularly limited as long as it can emit light having a wavelength of 300 nm to 400 nm, and a commercially available product or the like can be used.

Specifically, those commercially available as so-called 365 nm UV LEDs or 375 nm UV LEDs can be used without any particular limitation, and specific examples include the following ones.
Product name "SV16 Small 5W Ultraviolet Black Light Wavelength: 365 nm, USB Rechargeable UV LED Light", manufactured by Alonefire;
Portable Small Black Light Key Holder for Glowing Original Puzzle (UV-LED375-nano) manufactured by NICHIA CORPORATION, product name "Single-Bulb LED Black Light", and the like

### [Other Members]

In addition to the above-described chromatographic body and light source, the detection kit according to the present invention can be used in combination with various other members without departing from the scope of the present invention.

### [Fluorescent Color Level Sample]

As the other member, for example, a fluorescent color level sample can be included. The fluorescent color level sample is a fluorescent color level sample that enable the detection kit according to the present embodiment to carry out not only qualitative analysis but also quantitative analysis. This fluorescent color level sample is constituted by causing a detection target to undergo each chromatographic fluorescence depending on a plurality of concentrations thereof, preparing membranes (those having various fluorescence intensities that depending on the concentrations) where the fluorescence has occurred, creating fluorescent color samples corresponding to the plurality of concentrations, and then assembling them. That is, the fluorescent color level sample includes fluorescent color samples (a plurality of color samples having various fluorescent intensities that depending on the concentrations) corresponding to the plurality of concentrations of the detection target. It is freely determined according to the detection target how many kinds of the plurality of concentrations are prepared at what degrees of concentration differences; however, it is generally preferable to prepare about 10 to 20 kinds, since this contributes to quantitative determination.

By providing a fluorescent color level sample in this manner, quantification is possible by visually observing and confirming the color level of the fluorescent color level sample and the chromatographic fluorescent color and then confirming the concentration of the fluorescent color level sample having a color level closest to the fluorescent color of the chromatography, which makes it possible to constitute a detection kit that enables quantification.

As described above, the present invention makes it possible to realize "quantification" by "Visual inspection(observation)" by using a substance that generates fluorescence rather than a substance that emits light, preferably a fluorescent substance that absorbs light having a specific wavelength to emit light having a wavelength in another visible range.

### [Manufacturing Method]

The detection kit according to the present embodiment can be obtained by preparing a chromatographic body and a light source.

The chromatographic body according to the present embodiment can be obtained as follows.

The sample pad, the conjugate pad, the membrane, and the absorption pad are formed according to the conventional methods and are overlapped in an embodiment illustrated in Fig. 1.

Next, an antibody for detection is applied in a line shape onto the membrane and dried to form the test line 15a and the control line 15b. It is noted that, as the antibody, an antibody capable of being detected in this type of chromatography, such as an antibody SARS-CoV-2 Nucleoprotein antibody, an anti-HA antibody, or an anti-mouse antibody, can be used as the detection target without any particular limitation. In addition, another substance for detection other than the above antibodies can also be used.

Then, a detection marker containing a detection antibody bound to a fluorescent substance such as a europium complex is applied onto the conjugate pad and dried to form a conjugate pad on which the fluorescent substance is supported, thereby capable of forming the chromatographic body.

Further, a plurality of such chromatographic bodies are prepared, and a specimen solution adjusted to a plurality of concentrations is dropped onto each chromatographic body to carry out immunochromatography. Then, after a predetermined time (10 to 30 minutes) has elapsed, light is irradiated from the light source, and the emitted light was photographed. As such a plurality of emitted lights were photographed, And resultant photographic images of such a plurality of emitted lights are assembled to create a fluorescent color level sample.

### [Usage and Effects]

The detection kit according to the present embodiment can be used as illustrated in Fig. 2. It is noted that, for description, the fluorescent substance 30 is illustrated in a schematic manner, and the specimen and the detection target are also illustrated schematically in Fig. 2.

As illustrated in Fig. 2, in a case of using the detection kit 1 according to the present embodiment, first, a specimen 31 is dropped onto the sample pad 11. The specimen 31 migrates from the conjugate pad 13 to the membrane 15 by capillary action. In this case, a detection target 33 in the specimen 31 reacts with the detection marker (not illustrated for simplicity of the figure) in which the fluorescent substance 30 supported on the conjugate pad 13 is bound to the detection antibody, and migrates to the membrane 15 in a state of being bound to the detection marker.

Then, a binding product between the fluorescent substance 30 and the detection target 33 reacts with an antibody that constitutes the test line 15a and then remains at the test line 15a.

Then, detection can be carried out by emitting light having a predetermined wavelength from the light source 20.

In addition, in this case, the concentration of the detection target 33 can be measured through visual inspection(observation) by preparing a fluorescent color level sample and comparing the color with that of the fluorescent color level sample.

In a case of carrying out this detection operation, the desired effect of the present invention can be more effectively obtained by carrying out the operation in a dark place, for example, by using a dark box (using the detection kit according to the present invention).

The detection kit according to the present embodiment is constituted as described above, and thus the detection by visual inspection(observation) is possible by emitting light from a light source. Further, the Stokes shift is wide, there is no background influence, and it is easy to carry out the determination by visual inspection(observation). Further, because the discrimination is possible to an extent that allows for sufficient determination even with a small amount of the detection target, it can be said that the sensitivity has been improved, and the detection is possible even in a case where the target substance to be detected is small, for example, in the early stage of infection. In addition, confidentiality is high since confirmation cannot be carried out in a case where light having a specific wavelength is not emitted, and it is also possible to protect privacy by carrying out confirmation only when necessary. As a result, it is useful not only for simple test and detection of infectious diseases (diagnostic agents for infectious diseases), but also for test of pregnancy test drugs and allergic substances or drugs.

It is noted that the present invention is not limited to the above-described embodiment, and various modifications can be made without departing from the scope of the present invention.

### Examples

### [Example 1]

Using Estapor (registered trademark) microspheres (K1-050, F1-Eu-010) as a fluorescent substance, the detection kit illustrated in Fig. 1 was obtained in accordance with the above-described manufacturing method.

The raw materials and light source used are shown below.
Sample pad: Product name "CF4" (manufactured by Cytiva)
Conjugate pad: Product name "33glass" (manufactured by Cytiva)
Membrane: Product name "FF80 HP" (manufactured by Cytiva)
Absorption pad: Product name "SureWick (registered trademark)" Cellulose Sample Pad (C083 type, manufactured by Merck KGaA)
Microsphere concentration: Detection antibody (Anti-H5 HA monoclonal antibody (5H7)):particle = 1:10, particle equivalent: 0.025%, 10 µL
Light source: Product name "SV16 Small 5W Ultraviolet Black Light Wavelength: 365 nm", manufactured by Alonefire
Using the obtained detection kit, an H5 subtype avian influenza antigen (hemagglutinin) was detected.

For the detection, five antigen solutions were prepared at concentrations of 0, 1, 10, 100, and 1,000 (unit: ng/mL), and 0.1 mL was dropped onto each test kit (the actual amount used is shown in the figure) to carry out a test.

The results are shown in Fig. 3. In addition, in Fig. 3, simply colored samples prepared as the samples and are collectively shown.

As is revealed from the results shown in Fig. 3, it can be seen that the detection kit according to the present invention makes it possible to carry out sufficient detection at a low concentration of the antigen solution as low as a concentration of 1 ng/mL, and thus has high sensitivity. In addition, in a case where light is emitted from the light source, luminescence (red luminescence) is observed on the test line, which can be easily discriminated by visual inspection(observation), and thus detection can be carried out. Although not illustrated in the drawing, nothing is displayed unless light is emitted from a light source, which enables easy use even in a case where confidentiality is high. In addition, since the luminescence level differs depending on the antigen concentration in the antigen solution, it is also possible to carry out, although being simple, quantitative detection by carrying out a comparison with a level sample.

### [Example 2]

A detection kit having the configuration illustrated in Fig. 1 was obtained in the same manner as in Example 1, except that Estapor (registered trademark) microspheres (product number "F1-Eu-030") were used as a fluorescent substance, and the following raw materials were used in accordance with the following method.

### [Raw Material]

Sample pad: Product name "CF3" (manufactured by Cytiva)
Conjugate pad: Product name "33glass" (manufactured by Cytiva)
Membrane: Product name "Hi-Flow^{™} Plus HF12002" (manufactured by Merck KGaA)
Absorption pad: Product name "SureWick (registered trademark)" Cellulose Sample Pad (C083 type, manufactured by Merck KGaA)
Immobilized antibody; Anti-H5 HA monoclonal antibody (8C1), Anti-mouse IgG
Detection marker; Fluorescent substance "F1-Eu-030", Detection antibody: Anti-H5 HA monoclonal antibody (5H7)
Spreading solution; 50 mM Tris-HCl, 0.05% Tween 20, 0.5% Casein-Na, 10 mM Guanidine
Antigen; Influenza A H5N1 (A/Anhui/1/2005) HA Protein, His Tag (Sino Biological)

### [Other Materials]

Activation/Coupling Buffer - 50 mM MES, pH 6.0
EDC (F024810, Wako)
Sulfo NHS (24510, Thermo Scientific)
Ethanolamine (411000, Sigma)
Blocking Buffer - 10 mM Tris, pH 8.0 + 2% (w/v) BSA
Micro BCA [registered trademark] Protein Assay Kit (23235, Thermo Scientific)
   [Light Source] Product name "SV16 Small 5W Ultraviolet Black Light Wavelength: 365 nm", manufactured by Alonefire

### [Method]

In accordance with Merck KGaA's Application Note (Microsphere Coupling - Two-step EDC/Sulfo NHS Covalent Coupling Procedure for Estapor (registered trademark) Carboxyl-modified Dyed Microspheres), six types of detection markers were obtained by binding particles as a fluorescent substance to the detection antibody at concentrations of 0, 20, 40, 60, 80, and 100 (mg/g) using the detection antibody/particles (particles as a fluorescent substance). For each detection marker, the amount of detection antibody bound per 0.1 mg of particles was calculated by quantifying the amount of detection antibody remaining in the supernatant after the binding reaction between the detection antibody and the fluorescent substance according to the BCA method and subtracting it from the amount of detection antibody added. The data for each detection marker are shown in Table 1. It is noted that, in Table 1, the detection antibody is simply referred to as "antibody." The same applies to Table 2. The immobilized antibody was applied onto each of the test line and control line of the membrane, followed by blocking.

The obtained detection marker was applied onto the conjugate pad at a particle amount of 0.5 to 4 µg/pad and dried to prepare the test kit illustrated in Fig. 1. Then, a photographic image before dropping was captured (using SV16 Black Light, iPhone (registered trademark), camera application, x5). 80 µL of a spreading solution (antigen (+) indicates having an antigen in the spreading solution, and antigen (-) indicates having no antigen in the spreading solution; antigen amount is 1.25 ng/mL) was dropped thereon, and after 30 minutes, light was applied from a light source to take a photo of fluorescence. The results are shown in Fig. 4. In addition, the fluorescence intensity is shown in Fig. 6(a). The fluorescence of the detection marker and the background of the membrane were confirmed. As is revealed from the results shown in Fig. 4, it can be seen that, at 100, it is not very preferable from the fact that the background emits fluorescence, thereby being inferior in visibility by visual inspection(observation) and the economic viewpoint in terms of the fact that the advantage of increased concentration, such as further improved visibility, is not obtained (see Fig. 6(a)). In addition, at 20, it is inferior in visibility since the background emits fluorescence in the antigen (+), and at 80, it is inferior in visibility since the background emits fluorescence, although the fluorescence is faintly observed, and it is inferior in terms of the fact that the line fluorescence is blurred.

In addition, the concentration of the detection marker was set to satisfy detection antibody/particle (particle as fluorescent substance) = 80 (mg/g), the amount of particles was set to 0.5, 1, 2, and 4, and the relationship between the amount of particles as a fluorescent substance and the fluorescence was confirmed. The results are shown in Fig. 5. In addition, the fluorescence intensity is shown in Fig. 6(b). It can be seen that, as shown in Fig. 6(b), although the fluorescence intensity is improved in a case where the amount is increased, the background emits fluorescence in a case where the amount is increased, as shown in Fig. 5, and thus it is inferior in visibility by visual inspection(observation).

**[Table 1]**

| antibody/ particle ratio (mg/ g) | the amount of particles (µg) | the amount of antibody remaining in the supernatant (ug) | the amount of antibody bound particles (ng) | binding rario(%) |
|---|---|---|---|---|
| 0 | 0 | 0.07 | -0.07 | - |
| 20 | 2 | 0.12 | 1.88 | 93.80 |
| 40 | 4 | 0.10 | 3.90 | 97.52 |
| 60 | 6 | 0.13 | 5.87 | 97.88 |
| 80 | 8 | 0.26 | 7.74 | 96.70 |
| 100 | 10 | 1.33 | 8.67 | 86.74 |

### [Example 3]

Antigen detection was carried out in the same manner as in Example 2, except that the fluorescent substance was changed to Estapor (registered trademark) microspheres (F1-Eu-050).

The results are shown in [Table 2] and Figs. 7 to 9.

As is revealed from the results shown in Fig. 7, it can be seen that favorable results are obtained at 20 to 80, and favorable results are obtained, particularly at 40 to 60, in both cases of (-) and (+). In addition, in the same manner as in Example 2 even regarding the amount of particles, it can be seen that, although the fluorescence intensity is improved in a case where the amount is increased, the background emits fluorescence in a case where the amount is increased, and thus it is inferior in visibility by visual
inspection(observation).

**[Table 2]**

| antibody/ particle ratio (mg/ g) | the amount of particles (µg) | the amount of antibody remaining in the supernatant (ug) | the amount of antibody bound particles (ng) | binding rario(%) |
|---|---|---|---|---|
| 0 | 0 | 0.07 | -0.07 | - |
| 20 | 2 | 0.04 | 1.96 | 98.19 |
| 40 | 4 | 0.43 | 3.57 | 89.26 |
| 60 | 6 | 1.13 | 4.87 | 81.15 |
| 80 | 8 | 2.91 | 5.09 | 63.68 |
| 100 | 10 | 4.68 | 5.34 | 53.39 |

## Claims

1. A test kit comprising:
a sample pad configured to absorb a specimen;
a conjugate pad supporting a detection marker;
a chromatographic body comprising a membrane;
an absorption pad configured to prevent backflow of the specimen; and
a light source configured to emit light of 300 nm to 400 nm,
wherein the detection marker contains a fluorescent substance that emits light having a wavelength of 600 nm or more when irradiated with light having a wavelength of 300 nm to 400 nm.

2. The test kit according to claim 1, wherein the fluorescent substance is a rare earth complex.

3. The test kit according to claim 1, wherein the fluorescent substance has a fluorescence brightness of 0.8 or more when a brightness of an excitation light is set to 1.

4. The test kit according to claim 1, further comprising:
a fluorescent color level sample,
wherein the test kit is configured to allow quantification by visually comparing a color level of the fluorescent color level sample with a chromatographic fluorescent color on the chromatographic body.

5. The test kit according to claim 2, wherein the fluorescent substance is a polymer particle encapsulating t the rare earth complex.

6. The test kit according to claim 5, wherein a mixing ratio of the detection antibody to the polymer particle, which is the antibody/the particle (mg/g), in the detection marker is 20 to 80.
